(19) 

Europäisches Patentamt
European Patent Office
Office européen des brevets

(11) **EP 1 692 185 B1**

(12) **FASCICULE DE BREVET EUROPEEN**

(45) Date de publication et mention
de la délivrance du brevet:
**30.07.2008 Bulletin 2008/31**

(21) Numéro de dépôt: **04805570.1**

(22) Date de dépôt: **26.11.2004**

(51) Int Cl.:
*C07K 16/40* (2006.01)      *C12Q 1/48* (2006.01)
*G01N 33/573* (2006.01)

(86) Numéro de dépôt international:
**PCT/FR2004/003042**

(87) Numéro de publication internationale:
**WO 2005/054499 (16.06.2005 Gazette 2005/24)**

(54) **UTILISATION DE LA L-ISOASPARTYL METHYLTRANSFERASE COMME MARQUEUR DE LONGEVITE DES SEMENCES**

VERWENDUNG VON L-ISOASPARTYL-METHYLTRANSFERASE ALS LANGLEBIGKEITSMARKER IN SAATGUT

USE OF L-ISOASPARTYL METHYLTRANSFERASE AS LONGEVITY MARKER IN SEEDS

(84) Etats contractants désignés:
**AT BE BG CH CY CZ DE DK EE ES FI FR GB GR HU IE IS IT LI LU MC NL PL PT RO SE SI SK TR**

(30) Priorité: **26.11.2003 FR 0313858**

(43) Date de publication de la demande:
**23.08.2006 Bulletin 2006/34**

(73) Titulaire: **INSTITUT NATIONAL DE LA RECHERCHE AGRONOMIQUE (INRA)
75007 Paris Cédex 07 (FR)**

(72) Inventeurs:
• **GRAPPIN, Philippe
F-78370 Plaisir (FR)**
• **OGE, Laurent
F-92100 Boulogne (FR)**
• **BOVE, Jérome
F-02220 Braine (FR)**

(74) Mandataire: **Vialle-Presles, Marie José et al
Cabinet ORES,
36,rue de St Pétersbourg
75008 Paris (FR)**

(56) Documents cités:
**WO-A-96/12797          WO-A-96/15249
WO-A-98/15647          WO-A-03/057204**

• **MUDGETT MB ET AL.: "Protein Repair L-Isoaspartyl Methyltransferase in Plants" PLANT PHYSIOLOGY, vol. 115, no. 4, décembre 1997 (1997-12), pages 1481-1489, XP002285622 ROCKVILLE cité dans la demande**
• **THAPAR N ET AL.: "Distinct Patterns of Expression But Similar Biochemical Properties of Protein L-Isoaspartyl Methyltransferase in Higher Plants" PLANT PHYSIOLOGY, vol. 125, no. 2, février 2001 (2001-02), pages 1023-1035, XP002285623 ROCKVILLE**

SEQUENCE LISTING

<110> INSTITUT NATIONAL DE LA RECHERCHE AGRONOMIQUE (INRA)
GRAPPIN, Philippe
OGE, Laurent
BOVE, Jérome

<120> UTILISATION DE LA L-ISOASPARTYL METHYLTRANSFERASE COMME MARQUEUR DE LONGEVITE DES SEMENCES

<130> MJPbv539/118

<150> FR 0313858
<151> 2003-11-26

<160> 17

<170> PatentIn version 3.1

<210> 1
<211> 13
<212> PRT
<213> Artificial sequence

<220>
<223> Séquence consensus L-isoaspartyl méthyltransférases végétales

<220>
<221> MISC_FEATURE
<222> (9)..(9)
<223> X= E, V ou S

<220>
<221> MISC_FEATURE
<222> (10)..(10)
<223> X= A ou E

<220>
<221> MISC_FEATURE
<222> (13)..(13)
<223> X= R, G ou Q

<400> 1

```
Arg Tyr Val Pro Leu Thr Ser Arg Xaa Xaa Gln Leu Xaa
 1               5                   10
```

<210> 2
<211> 16
<212> PRT
<213> Artificial sequence

<220>
<223> Séquence consensus L-isoaspartyl méthyltransférases végétales

<220>
<221> MISC_FEATURE
<222> (2)..(2)
<223> X= D ou E

<220>
<221> MISC_FEATURE
<222> (4)..(4)
<223> X= Q ou K

<220>
<221> MISC_FEATURE
<222> (6)..(6)
<223> X= V ou I

<220>
<221> MISC_FEATURE
<222> (9)..(9)
<223> X= N ou S

<220>
<221> MISC_FEATURE
<222> (20)..(10)
<223> X= S, E ou A

<220>
<221> MISC_FEATURE
<222> (14)..(14)
<223> X= IS, VS, VT, TS ou une liaison peptidique

<220>
<221> MISC_FEATURE
<222> (15)..(15)
<223> X= I ou V

<220>
<221> MISC_FEATURE
<222> (16)..(16)
<223> X= K, Q ou R

<400> 2

```
Gln Xaa Leu Xaa Val Xaa Asp Lys Xaa Xaa Asp Gly Ser Xaa Xaa Xaa
 1           5                   10                  15
```

<210> 3
<211> 17
<212> PRT
<213> Arabidopsis thaliana

<400> 3

Gln Asp Leu Gln Val Val Asp Lys Asn Ser Asp Gly Ser Val Ser Ile
1               5                   10                  15

<210> 4
<211> 15
<212> PRT
<213> Arabidopsis thaliana

<400> 4

Gln Glu Leu Lys Val Ile Asp Lys Asn Glu Asp Gly Ser Ile Lys
1               5                   10                  15

<210> 5
<211> 13
<212> PRT
<213> Arabidopsis thaliana

<400> 5

Arg Tyr Val Pro Leu Thr Ser Arg Glu Ala Gln Leu Arg
1               5                   10

<210> 6
<211> 13
<212> PRT
<213> Arabidopsis thaliana

<400> 6

Arg Tyr Val Pro Leu Thr Ser Arg Val Glu Gln Leu Gly
1               5                   10

<210> 7
<211> 13
<212> PRT
<213> Arabidopsis thaliana

<400> 7

Arg Tyr Val Pro Leu Thr Ser Arg Ser Ala Gln Leu Gln
1               5                   10

<21d> 8
<211> 17
<212> PRT

<213> Arabidopsis thaliana

<400> 8

```
Gln Asp Leu Gln Val Ile Asp Lys Ser Ala Asp Gly Ser Thr Ser Val
1               5               10                  15
```

<210> 9
<211> 17
<212> PRT
<213> Arabidopsis thaliana

<400> 9

```
Gln Glu Leu Gln Val Val Asp Lys Asn Ala Asp Gly Ser Val Thr Val
1               5               10                  15

Gln
```

<210> 10
<211> 8
<212> PRT
<213> Arabidopsis thaliana

<400> 10

```
                        Arg Tyr Val Pro Leu Thr Ser Arg
                        1               5
```

<210> 11
<211> 12
<212> PRT
<213> Arabidopsis thaliana

<400> 11

```
Arg Tyr Val Pro Leu Thr Ser Arg Glu Ala Gln Leu
1               5               10
```

<210> 12
<211> 15
<212> PRT
<213> Arabidopsis thaliana

<400> 12

```
Arg Tyr Val Pro Leu Thr Ser Arg Glu Ala Gln Leu Arg Gly Asp
1               5               10                  15
```

<210> 13
<211> 23
<212> DNA
<213> Arabidopsis thaliana

<400> 13
gctatggagg ctgtggatag agg          23


<210> 14
<211> 21
<212> DNA
<213> Arabidopsis thaliana


<400> 14
tcagtcccct ctcagctgcg c          21


<210> 15
<211> 21
<212> DNA
<213> Arabidopsis thaliana


<400> 15
ggaccgggta cttaactgct t          21


<210> 16
<211> 24
<212> DNA
<213> Arabidopsis thaliana


<400> 16
ttggcggcac ccttagctgg atca          24


<210> 17
<211> 25
<212> DNA
<213> Arabidopsis thaliana


<400> 17
atgccccagg acatcgtgat ttcat          25


## Description

La présente invention est relative à l'utilisation de la protéine L-isoaspartyl méthyltransférase comme marqueur du vieillissement de lots de semences.

Le vieillissement des semences, qui intervient au cours de leur stockage, s'accompagne de la détérioration des membranes cellulaires, de la modification de l'activité catalytique de nombreuses enzymes (pour revue cf. WALTERS C., 1998; Seed Sci. Res., 8: 223-244), et de l'accumulation de mutations de l'ADN. L'ensemble de ces phénomènes entraîne une diminution de la vigueur germinative et de la viabilité des semences.

La rapidité du vieillissement dépend d'un ensemble complexe de facteurs intrinsèques (notamment facteurs génétiques) et extrinsèques (conditions de conservation). Comme le degré de vieillissement conditionne la qualité germinative des semences, ainsi que leur aptitude à une conservation ultérieure, il est souhaitable de pouvoir le déterminer le plus rapidement et le plus précisément possible.

Les tests actuellement disponibles pour évaluer la qualité germinative d'un lot de semences sont principalement des tests basés sur des essais de germination prenant en compte différents paramètres, tels que le délai moyen de germination du lot, l'étalement de la germination dans le temps, la capacité germinative, et la fréquence de plantules normales. La faculté germinative est par exemple définie par le pourcentage de plantules normales ayant germées, au bout d'un nombre de jours donné après le semis, pondéré par le pourcentage de très jeunes pousses. Ces tests sont définis et calibrés pour chaque espèce ou variété par un organisme de certification des semences selon les normes ISTA (International Seed Testing Association). Cependant, ces essais sont lourds à mettre en oeuvre. D'autre part ils permettent d'évaluer la qualité germinative actuelle d'un lot de semences, mais pas de prédire son évolution future, et notamment ne permettent en aucun cas d'anticiper une chute de vigueur germinative.

On ne dispose pas à l'heure actuelle, de marqueurs spécifiques permettant de prédire simplement et avec précision la viabilité d'un lot de semences, et *a fortiori* sa capacité de conservation.

Parmi les multiples enzymes dont l'activité apparaît modifiée au cours du vieillissement (cf. WALTERS C, 1998, Seed Sci. Res., 8: 223-24 4 précité), les Inventeurs se sont plus particulièrement intéressés à la L-isoaspartyl méthyltransférase (IAMT).

La L-isoaspartyl méthyltransférase (EC 2.1.1.77) est une enzyme de réparation protéique qui intervient dans la conversion de résidus anormaux L-isoaspartate et D-aspartate présents dans les protéines, en résidus L-aspartate.

Les résidus L-isoaspartate et D-aspartate qui constituent le substrat de la L-isoaspartyl méthyltransférase résultent de la déamidation, de l'isomérisation et de la racémisation spontanées de résidus aspartate et asparagine, qui interviennent au cours du vieillissement des graines, et qui font partie des modifications pouvant affecter la fonctionnalité des protéines dans lesquelles ces résidus sont intégrés.

La L-isoaspartyl méthyltransférase (IAMT ou PIMT) permet de convertir au moins une partie de ces résidus en résidus L-aspartate, permettant aux protéines ainsi réparées de retrouver leur activité.

La L-isoaspartyl méthyltransférase est une enzyme quasi universelle, que l'on retrouve dans de très nombreux organismes, depuis les bactéries (à gram négatif), jusqu'aux plantes et animaux (O'CONNER C.M et al, 1985; Biochem. Biophys. Res., 132: 1144-1150).

Chez les plantes, l'activité L-isoaspartyl méthyltransférase apparaît présente dans la quasi-totalité des espèces végétales ; l'activité L-isoaspartyl méthyltransférase a notamment été mise en évidence dans le germe de blé (TRIVEDI L. et al., Eur. J. Biochem., 1982, 128: 349-354, MUDGETT M.B. et al, Biochemistry, 1993, 32: 11100-11111, J. Biol. Chem., 1994, 269(41): 25605-25612) puis chez le lotus (SHEN-MILLER et al., Am. J. Bot., 1995, 82(11): 1367-1380), *Arabidopsis thaliana* (MUDGETT M.B. et al, Plant. Mol. Biol., 1996, 30: 723-737 ; THAPAR N. et al, Protein Expr. Purif., 2000, 20: 237-251), l'orge (MUDGETT M.B. et al., Plant Physiol., 1997, 115: 1481-1489), la tomate (KESTER et al., J. Exp. Bot., 1997, 309: 943-949), le maïs, la carotte et le riz (THAPAR N. et al., Plant Physiol., 2001, 125(2): 1023-1035).

Cette activité est détectable dans de nombreux organes chez les plantes, mais elle apparaît maximale dans les graines sèches (MUDGETT M.B. et al., Plant. Mol. Biol., 1996, 30: 723-737). CLARKE S. (Int. J. Pept. Protein Res., 1987, 30: 808-821) a montré *in vitro* que la IAMT a une activité de réparation d'environ 30% des résidus anormaux ; son bon fonctionnement permettrait donc à la graine ou à la plante de ralentir la perte d'intégrité des protéines accumulant des acides aminés L-isoaspartate, mais pas de compenser en totalité les phénomènes de racémisation et d'isomérisation.

SHEN-MILLER et al. (Am. J. Bot., 1995, 82(11): 1367-1380) ont dosé l'activité IAMT dans des graines de Lotus Sacré *(Nelumbo nucifera)* âgées d'environ 90 ans. Les graines qui se sont révélées capables de germer possédaient une activité IAMT élevée et le taux de résidus racémisés était équivalent à celui de graines fraîches, soit 2%. KESTER et al., (J. Exp. Bot., 1997, 309: 943-949) ont rapporté, chez des graines de tomates vieillies artificiellement, une diminution de l'activité IAMT corrélée à une baisse de la capacité germinative. MUDGETT M.B. et al., (Plant Physiol., 1997, 115: 1481-1489), ont également observé, sur des graines d'orge vieillies naturellement, que l'activité L-isoaspartyl méthyltransférase diminuait en fonction de l'âge des semences et que cette diminution s'accompagnait de l'accumulation de résidus L-isoaspartyl racémisés ainsi que d'une perte de viabilité des semences ; en revanche, dans le cas des graines d'orge vieillies artificiellement, ils ont aussi observé une accumulation de résidus racémisés et une diminution de la capacité germinative, mais pas de baisse de l'activité IAMT.

Ces travaux ne permettent toutefois pas de conclure à l'existence d'une corrélation directe entre la quantité de L-isoaspartyl méthyltransférase et la capacité germinative des graines, qui permettrait d'utiliser cette protéine comme marqueur de la capacité germinative.

En outre, les résultats décrits dans les travaux mentionnés ci-dessus s'appuient soit sur des tests d'activité IAMT, soit sur la détection de l'ARNm de la L-isoaspartyl méthyltransférase.

Or, le dosage de l'activité enzymatique fait appel à des méthodes lourdes et nécessitant l'utilisation de méthanol radioactif, qui sont difficiles à mettre en oeuvre dans le cadre de contrôle de routine. D'autre part, en ce qui concerne l'ARNm, il a été observé qu'il n'y avait pas toujours de corrélation entre la détection de transcrits de l'IAMT et l'activité enzymatique IAMT. Ainsi, MUDGETT M.B. et al (Plant. Mol. Biol., 1996, 30: 723-737) ont rapporté que chez *Arabidopsis thaliana,* l'ARNm de l'IAMT était détectable dans les plantules (où l'activité enzymatique n'est pas détectable) mais pas dans la graine sèche (où l'activité enzymatique est maximale).

Chez *Arabidopsis thaliana,* deux gènes codant pour des L-isoaspartyl méthyltransférases dont l'activité a été montrée *in vitro* ont été identifiés à l'heure actuelle. Le premier d'entre eux a été décrit par MUDGETT. *et al* (1996, précité) ; le second a été décrit par XU Q. et al. (Plant Physiol., 2004, 136 :1-13) ; ces deux gènes ont été dénommés par cette dernière équipe *PIMT-1* et *PIMT-2,* et leurs produits de traduction ont été respectivement dénommés PIMT-1 et PIMT-2.

Ces deux gènes *PIMT-1* et *PIMT-2* sont respectivement situés sur les chromosomes 3 et 5 du génome d'*Arabidopsis*. Mais il n'a pas été décrit à l'heure actuelle quelle est la part de ces deux gènes dans les activités enzymatiques mesurées *in vivo.*

Les Inventeurs sont parvenus à identifier un mutant de régulation du gène *PIMT-1* chez *Arabidopsis thaliana* et ont ainsi pu constater que l'expression de ce gène joue un rôle essentiel dans la capacité de conservation de la graine sèche au cours du stockage. Ils ont en outre identifié, à partir de la protéine PIMT-1, un peptide permettant d'obtenir des anticorps capables de différencier PIMT-1 de PIMT-2 chez *Arabidopsis thaliana,* et de reconnaître des orthologues de PIMT-1 chez d'autres dicotylédones, et un peptide permettant d'obtenir des anticorps reconnaissant des orthologues de PIMT-1 chez d'autres espèces végétales, dicotylédones ou monocotylédones.

La quantification de L-isoaspartyl méthyltransférase à l'aide d'anticorps obtenus contre l'un ou l'autre de ces peptides a permis d'établir une corrélation entre la disparition de la protéine dans la graine sèche et la perte de vigueur germinative. En outre, les Inventeurs ont observé que la diminution de la quantité de L-isoaspartyl méthyltransférase précède la baisse de vigueur germinative. Il apparaît donc que cette enzyme constitue un marqueur prédictif, qui permet non seulement d'évaluer la capacité germinative des semences, mais également de prédire une baisse de cette capacité au cours de la conservation.

La présente invention a pour objet un procédé de détermination de la vigueur germinative et/ou de l'aptitude d'un lot de semences à la conservation, caractérisé en ce qu'il comprend la quantification, sur un échantillon de semences prélevé sur ledit lot, des protéines reconnues par des anticorps anti-L-isoaspartyl méthyltransférase dirigés contre une région de ladite protéine définie par la séquence (I) : RYVPLTSRX$_1$X$_2$QLX$_3$ (SEQ ID NO : 1), dans laquelle X$_1$ représente E, V ou S, X$_2$ représente A ou E, X$_3$ représente R, G ou Q.

Q, D, G, S, V, R, Y, V, P, L, T, S, R, K, I, E, A, N, H, F ont ici leur signification usuelle en code 1 lettre.

Les anticorps dirigés contre un peptide de séquence (I) reconnaissent les L-isoaspartyl méthyltransférases PIMT-1 et PIMT-2 chez *Arabidopsis thaliana,* ainsi que des orthologues de ces protéines chez d'autres espèces végétales, dicotylédones ou monocotylédones.

A titre de comparaison, des anticorps dirigés contre une autre région de la L-isoaspartyl méthyltransférase, définie par la séquence (II) : QX$_4$LX$_5$VX$_6$DKX$_7$X$_8$DGSX$_9$X$_{10}$X$_{11}$ (SEQ ID NO : 2), dans laquelle X$_4$ représente D ou E, X$_5$ représente Q ou K, X$_6$ représente V ou I, X$_7$ représente N ou S, X$_8$ représente S, E, ou A, X$_9$ représente soit un dipeptide choisi parmi IS, VS, VT, et TS, soit une liaison peptidique, X$_{10}$ représente I ou V, X$_{11}$ représente K, Q ou R, (par exemple des anticorps dirigés contre les peptides SEQ ID NO: 3, 4, 8, ou 9) présentent une spécificité plus restreinte. Le procédé conforme à l'invention peut avantageusement être mis en oeuvre par dosage immunologique, en utilisant des anticorps anti-L-isoaspartyl méthyltransférase dirigés contre un peptide de séquence (I).

La présente invention a également pour objet tout anticorps anti-isoaspartyl méthyltransférase dirigé contre une région de ladite protéine-définie par la séquence (I) ci-dessus.

A titre d'exemples non-limitatifs d'anticorps anti-isoaspartyl méthyltransférase dirigés contre une région définie par la séquence (I), on peut citer : des anticorps dirigés contre la région de séquence RYVPLTSREAQLR (SEQ ID NO : 5) de la PIMT-1 d'*Arabidopsis thaliana ;* des anticorps dirigés contre la région de séquence RYVPLTSRVEQLG (SEQ ID NO: 6) de la PIMT-2 d'*Arabidopsis thaliana* ; des anticorps dirigés contre la région de séquence RYVPLTSRSAQLQ (SEQ ID NO : 7) d'une PIMT de blé ; des anticorps dirigés contre la région de séquence RYVPLTSRSAQLQ (SEQ ID NO : 7) d'une PIMT de riz.

On définit ici comme anticorps dirigé contre une région d'une L-isoaspartyl méthyltransférase tout anticorps capable de se lier de manière détectable avec la région concernée, sur la protéine entière ou sur un fragment de celle-ci, mais ne se liant pas de manière détectable avec une autre région de la même protéine. Dans le cas d'anticorps monoclonaux, ceci inclut tout anticorps reconnaissant un épitope situé dans ladite région ; dans le cas d'anticorps polyclonaux, ceci inclut toute préparation d'anticorps ne présentant pas de réactions croisées significatives avec d'autres régions de la L-isoaspartyl méthyltransférase.

En revanche, cet anticorps peut présenter un pourcentage plus ou moins important de réactions croisées avec la région homologue de L-isoaspartyl méthyltransférases d'autres espèces végétales. Notamment, comme indiqué ci-dessus, des anticorps dirigés contre la région définie par la séquence (I) présentent généralement une spécificité très large et peuvent reconnaître une grande variété de L-isoaspartyl méthyltransférases chez les monocotylédones ou les dicotylédones.

Des anticorps conformes à l'invention peuvent être obtenus par des méthodes bien connues en elles-mêmes. Classiquement, on utilise comme immunogène un fragment de L-isoaspartyl méthyltransférase (sous forme d'un peptide naturel, recombinant, ou de synthèse) comprenant la région contre laquelle on souhaite diriger l'anticorps, ou au moins un fragment de celle-ci de taille suffisante pour constituer un épitope B (généralement au moins 5 à 7 acides aminés). Si nécessaire, ledit peptide est mélangé à un adjuvant, ou couplé à une protéine porteuse, afin d'augmenter son immunogénicité. Les anticorps obtenus peuvent ensuite être purifiés, de manière également connue en elle-même. Généralement, cette purification comprend au moins une étape de chromatographie d'affinité sur une colonne sur laquelle est greffée le peptide contre lequel doit être dirigé l'anticorps.

A titre d'exemples non-limitatifs, des anticorps dirigés contre la région d'une L-isoaspartyl méthyltransférase définie par la séquence (I) peuvent être obtenus en utilisant comme immunogène, et/ou pour la purification des l'anticorps par chromatographie d'affinité, le peptide de séquence RYVPLTSREAQLR (SEQ ID NO : 5), qui correspond à cette région dans la L-isoaspartyl méthyltransférase PIMT-1 d'*Arabidopsis thaliana* ; on peut également utiliser de la même manière des peptides comprenant un fragment de cette séquence, par exemple le fragment de séquence RYVPLTSR (SEQ ID NO : 10), ou le fragment de séquence RYVPLTSREAQL (SEQ ID NO : 11) ; on peut aussi utiliser des fragments plus long de PIMT-1, par exemple un peptide de séquence RYVPLTSREAQLRGD (SEQ ID NO : 12).

La présente invention a également pour objet un procédé de quantification de la L-isoaspartyl méthyltransférase dans du matériel végétal, caractérisé en ce qu'il comprend la mise en contact dudit matériel avec un anticorps anti-L-isoaspartyl méthyltransférase conforme à l'invention.

La présente invention a également pour objet l'utilisation d'un anticorps anti-L-isoaspartyl méthyltransférase conforme à l'invention pour déterminer la vigueur germinative et/ou l'aptitude à la conservation d'un lot de semences.

La présente invention sera mieux comprise à l'aide du complément de description qui va suivre, qui se réfère à des exemples décrivant la mise en évidence de la corrélation entre la quantité de L-isoaspartyl méthyltransférase et l'aptitude des graines à la conservation, ainsi que l'obtention d'un anticorps anti-L-isoaspartyl méthyltransférase et son utilisation pour évaluer la qualité germinative de semences de différentes espèces.

**EXEMPLE 1 : IDENTIFICATION ET CARACTERISATION D'UN MUTANT DE REGULATION DE LA L-ISOASPARTYL METHYLTRANSFERASE CHEZ *ARABIDOPSIS THALIANA***

Le criblage de la collection de transformants ADN-T d'*Arabidopsis thaliana* de l'INRA de Versailles, a permis d'isoler un mutant d'insertion au niveau du gène *PIMT-1*. Ce mutant a été dénommé atpcm1.1⁻. Les données de séquençage montrent que l'insertion ADN-T est localisée à 173 paires de bases en amont de l'ATG, dans la région promotrice du gène de la L-isoaspartyl méthyltransférase, où elle a causé une délétion de 13 paires de bases, faisant disparaître une boîte de réponse à l'acide abscissique (boîte ABRE pour Abscissic acid-Response Element)..

Des plantes mutantes homozygotes pour l'insertion de l'ADN-T ont été sélectionnées pour l'étude des propriétés du mutant.

**Expression du gène *PIMT-1* chez le mutant *atpcm1.1⁻***

L'expression du gène *PIMT-1* dans les plantules et dans les graines sèches chez le mutant *atpcm1.1⁻*, et chez la plante sauvage a été comparée par RT-PCR semi-quantitative. A titre de contrôle on utilise le gène du facteur de transcription EF qui présente un profil d'expression constitutif au cours du développement chez la graine d'*Arabidopsis thaliana*.

Les plantules sont obtenues par semis de graines sur un filtre de nylon (BUISINE, NYCOM, mailles de $5\mu$m de diamètre) reposant sur un milieu eau gélosée 0,5%. Après stratification (4˚C, 2 jours), les boîtes sont déposées en chambre de culture pendant 4 jours. Les filtres sur lesquels sont disposées les plantules, sont ensuite transférés sur un milieu eau gélosée pendant un jour, avant que les ARN soient extraits.

Les ARN totaux sont extraits des plantules ou des graines sèches selon la technique au phénol chaud de VERWOERD et al. (Nucleic Acids Res., 1989, 17(6): 2362), modifiée par l'ajout d'une seconde précipitation au LiCl (2 M final) de 6 heures. La purification des poly(A)⁺ est réalisée à partir des ARN totaux à l'aide d'oligo(dT)$_{25}$ fixés à des

billes magnétiques s Dynabeads® (DYNAL).

Le premier brin d'ADNc est synthétisé par rétrotranscription (RT) des ARNm poly(A)⁺.
L'amplification par PCR est réalisée sur 10 ng du produit de RT.

On utilise pour le gène de la L-isoaspartyl méthyltransférase, l'un des couples d'amorces suivants (spécifiques du gène *PIMT-1*) :

AMEAs:

GCT ATG GAG GCT GTG GAT AGA GG (SEQ ID NO: 13);

AQLRa :

TCA GTC CCC TCT CAG CTG CGC (SEQ ID NO: 14) ;

ou
GTGYs :

GGA CCG GGT ACT TAA CTG CTT (SEQ ID NO: 15) ;

et AQLRa
et pour le gène du facteur d'élongation EFα, le couple d'amorces suivant :

F1αA4-low :

TTG GCG GCA CCC TTA GCT GGA TCA (SEQ ID NO : 16) ;

F1αA4-up:

ATG CCC CAG GAC ATC GTG ATT TCA T (SEQ ID NO: 17) ;

La PCR est arrêtée à 15, 18 ou 21 cycles afin d'anticiper le moment où l'amplification n'est plus linéaire, donc plus interprétable en termes de quantités initiales.

Les produits de PCR sont déposés sur un gel d'agarose (0,8%) puis transférés, en milieu alcalin (soude 0,4 N) sur membrane de nylon, et cette dernière est hybridée avec une sonde radiomarquée spécifique du gène *PIMT* (sonde IAMT) ou avec une sonde radiomarquée spécifique du gène *EF*.

La quantification du signal d'hybridation est effectuée avec le logiciel MacBas®.

**Résultats obtenus dans les plantules de 5 jours :**

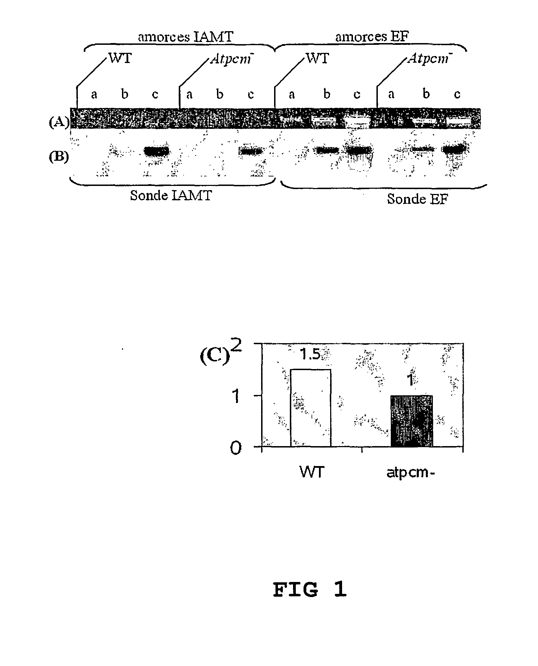

Ces résultats sont illustrés par la Figure 1.
Légende de la Figure 1 :

(A) Gel d'électrophorèse ;
(B) Autoradiographie après hybridation avec la sonde ;
(C) Quantification des signaux ;
WT : plantules sauvages ; *Atpcm* : plantules du mutant *atpcm1.1⁻*
a : 15 cycles de PCR ; b : 18 cycles de PCR ; c : 21 cycles de PCR

Ces résultats montrent que le gène s'exprime 1,5 fois plus chez le sauvage que chez le mutant au stade plantule de 5 jours sur eau gélosée.

Cette expérimentation a également été effectuée à partir de plantules qui, après un passage de 4 jours en chambre de culture ont été transférées sur un milieu eau gélosée contenant 100 μM d'acide abscissique, un jour avant l'extraction des ARN.

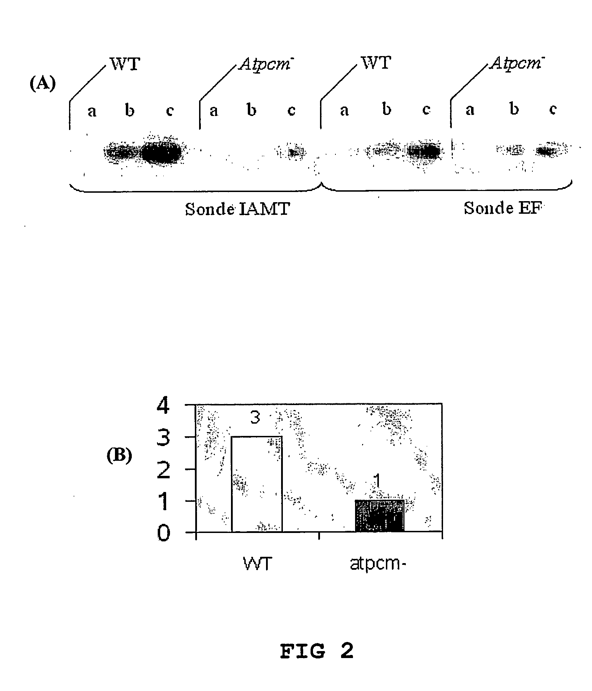

Les résultats sont illustrés par la Figure 2 :

Légende de la Figure 2 :

(A) Autoradiographie après hybridation avec la sonde ;

(B) Quantification des signaux ;
WT : plantules sauvages ; *Atpcm* : plantules du mutant *atpcin1.1⁻*
a : 15 cycles de PCR ; b : 18 cycles de PCR ; c : 21 cycles de PCR

Ces résultats montrent que les plantules sauvages surexpriment le gène *IAMT* en présence d'ABA, ce qui confirme les observations précédemment effectuées par MUDGETT M.B. et al (Plant. Mol. Biol., 1996, 30: 723-737, précité), et qu'elles accumulent 3 fois plus de transcrits IAMT que les plantules *atpcm1.1⁻*. Le mutant *atpcm1.1⁻* a visiblement perdu sa capacité d'induire l'expression de l'IAMT en présence d'ABA dans les plantules. Cette donnée permet de confirmer que la région délétée par la mutation contient bien une séquence régulatrice (boîte ABRE) impliquée dans la réponse à l'ABA

**Résultats obtenus dans les graines sèches :**

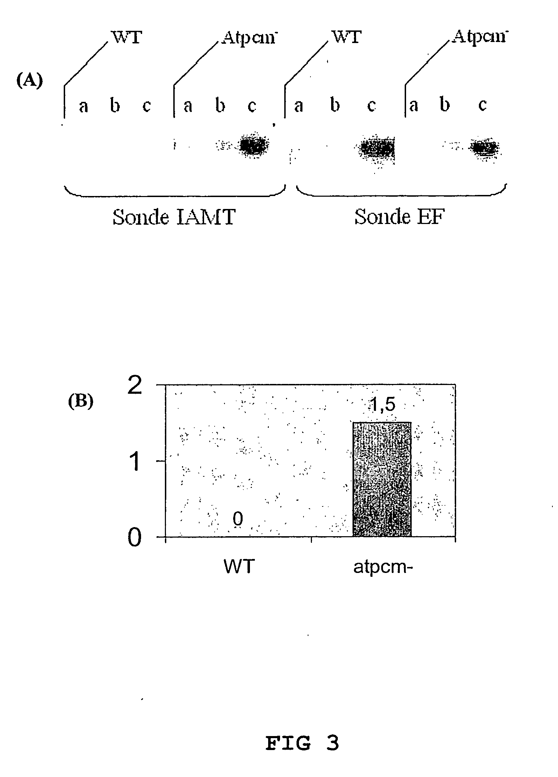

Les résultats sont illustrés par la Figure 3 :

Légende de la Figure 3 :

(A) Autoradiographie après hybridation avec la sonde;
(B) Quantification des signaux ;
WT : graines sauvages ; *Atpcm* : graines du mutant *atpcm1.1⁻*
a : 15 cycles de PCR ; b : 18 cycles de PCR ; c : 21 cycles de PCR.

On n'observe aucune accumulation des transcrits IAMT dans les graines sèches de la plante sauvage, ce qui confirme les observations précédemment effectuées par MUDGETT M.B. et al (Plant. Mol. Biol., 1996, 30: 723-737, précité). En revanche, le mutant *atpcm1.1⁻* accumule le transcrit IAMT à un niveau non négligeable dans la graine sèche.

**EXEMPLE 2 : INFLUENCE DU VIEILLISSEMENT ACCELERE SUR LA VIGUEUR GERMINATIVE DES GRAINES D'*ARABIDOPSIS THALIANA* DE TYPE SAUVAGE, OU DU MUTANT *ATPCM1.1-***

Des expérimentations de vieillissement accéléré ont été effectuées sur des graines fraîchement récoltées afin de tester s'il existe une différence de viabilité entre le type sauvage et le mutant *atpcm1.1⁻*. Les conditions de vieillissement choisies affectent très progressivement la vigueur germinative des semences pour ensuite conduire à une perte de viabilité. Le choix de conditions douces mime les conditions de vieillissement naturel et permet d'analyser finement l'impact de la IAMT dans l'évolution de la vigueur germinative au cours du vieillissement.

La production des semences du mutant et du témoin sauvage est réalisée en cultivant les plantes mères en même temps et dans la même enceinte. Le vieillissement accéléré est réalisé à 40°C (étuve Jouan) et 48% HR (obtenu avec le sel $Mg(NO_3)_2$ saturé) (bocal hermétique et graines dans sachets en papier filtre stériles). La température et l'humidité sont contrôlées avec Testostor 175 (Testo).

Des échantillons sont prélevés au bout de 2, 6 et 9 semaines de vieillissement et un test de germination est effectué sur 200 graines.

La germination se fait en boîte de Pétri (diamètre 55 mm) sur milieu aqueux additionné de MES (acide 2-N-Morpholino Ethane Sulfonique) à 0,58 g/l, ajusté à pH 5,9, et solidifié par l'agar (7g/l). Le semis est effectué sous hotte à flux laminaire, à raison de 25 graines par boîte, disposées régulièrement sur le milieu pour mieux repérer le début de la germination. Les graines semées sont observées quotidiennement à la loupe. Une graine est considérée comme germée lorsque la sortie de la radicule est visible à la loupe.

Chaque essai comprend 8 répétitions de 25 graines et les moyennes des pourcentages de graines germées après 3 jours d'imbibition sont reportées sur le graphe. Une graine est dite germée lorsque la radicule est visible.

La germination se fait en boîte de Pétri (diamètre 55mm), à raison de 25 graines par boîte, sur milieu aqueux solide (agar 7g/l) complété avec 0,58 g/l de MES (acide 2-N-Morpholino Ethane Sulfonique) et ajusté à pH 5,9.

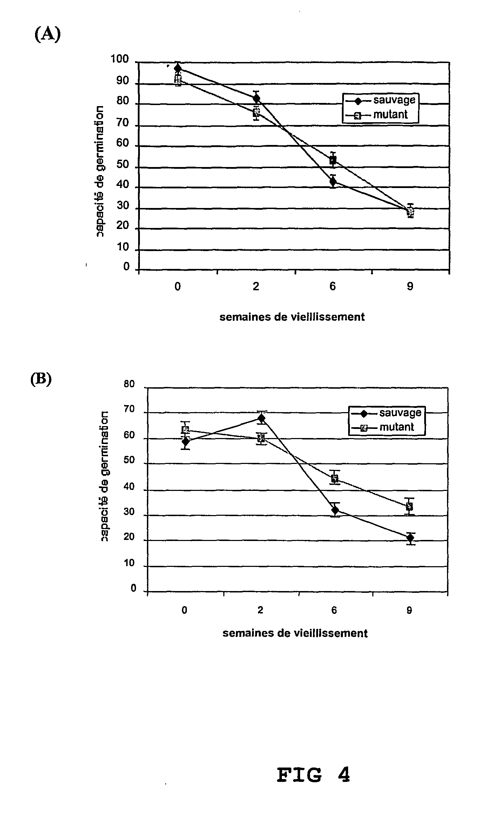

Ce test est effectué dans différentes conditions de température: (1) une alternance de températures 20-25°C, qui correspond aux conditions optimales pour la germination et permet de mesurer la viabilité des semences ; (2) une température de 15°C, qui est légèrement pénalisante pour la germination ; une baisse de la capacité germinative dans ces conditions reflète une baisse de vigueur germinative du lot de semences. Les résultats sont illustrés par la Figure 4. Légende de la Figure 4 : Capacité germinative 3 jours après semis, à 20-25°C (A), et 15°C (B), en fonction de la durée de stockage.

♦ : graines sauvages ;
■ : graines du mutant *atpcm1.1⁻*

La cinétique de germination effectuée avec une alternance de température 20-25˚C (Figure 4 A) ne montre pas de différences significatives dans la capacité de germination des graines du mutant et du type sauvage. En revanche, lorsque les graines sont imbibées à 15 ˚C (Figures 4 B), qui est une températures où des différences de vigueur germinative peuvent être mises en évidence, il apparaît que la vigueur germinative des graines de type mutant, à partir de 6 semaines de vieillissement accéléré, est supérieure à celle des graines de type sauvage d'environ 30%. Cette expérience a été reproduite 3 fois avec des échantillons biologiques indépendants et a conduit au même résultat.

## EXEMPLE 3 : PRODUCTION D'ANTICORPS ANTI -IAMT.

2 peptides de la région C-terminale de l'isoaspartyl méthyltransférase-1 d'*Arabidopsis thaliana* : QDLQVVDKNSDGSVSIK (peptide 1, SEQ ID NO : 3 , à titre de comparaison), et : RYVPLTSREAQLR (peptide 2, SEQ ID NO : 5) ont été choisis comme immunogènes.

Les anticorps ont été préparés chez le lapin selon le protocole suivant:

Après prélèvement de 1,5 ml de sérum « pré-immun », l'immunisation des lapins est effectuée par une première injection du peptide choisi (4 mg en solution dans 200 $\mu$g de peptide dans 5 ml d'une solution contenant la protéine porteuse KLH mélangé à 5 ml de l'adjuvant de Freund's, suivie d'injections de rappel 1, 2, 4, 8 et 15 semaines après la 1ère injection. Le sérum immun (50 ml) est prélevé 20 semaines après la 1ère injection.

Les anticorps sont purifiés par chromatographie d'affinité sur une colonne de SEPHAROSE 4F (PHARMACIA) activé au bromure de cyanogène, greffée avec 20 mg par gramme de gel SEPHAROSE du peptide utilisé comme immunogène.

5 ml de sérum préalablement dilué dans 5 ml de PBS 1x (NaC-140-mM ; KCl 2,5 mM ; $Na_2HPO_4$ 8,1 mM ; $KH_2 PO_4$ 15 mM ; pH 7,2) sont appliqués sur la colonne.

Le gel est lavé 3 fois par 10.ml de PBS 1x. L'élution est effectuée par passage de 10 ml d'une solution 0,2 M glycine/0,15 M Nacl pH2. Les fractions éluées sont collectées dans des tubes contenant 0,1 ml de Tris pH 8,8 (1,5 M)

Les fractions contenant l'anticorps sont rassemblées, (dialysées contre une solution de PBS 1x froid), puis complétées avec 0,02% d'azide de sodium. Les fractions sont conservées à 4˚C.

Pour vérifier la spécificité de l'anticorps contre la protéine PIMT-1 les ADNc des gènes *PIMT-1 et PIMT-2* ont été clonés séparément dans le vecteur d'expression pDEST17 (INVITROGEN) sous contrôle du promoteur bactérien T7 et en aval d'une étiquette (TAG) 6xHis, et exprimés dans la souche BL21 de *E. coli*.

Les protéines PIMT-1 et PIMT-2 recombinantes exprimées par *E. coli* ont été individuellement purifiées par chromatographie d'affinité sur billes de nickel, et ont été analysées par Western blot avec l'anticorps 1.

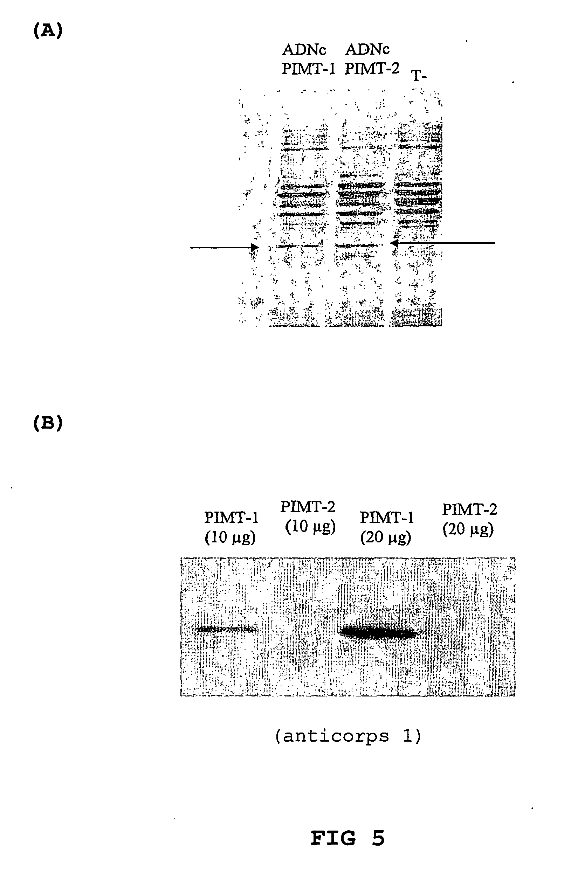

Les résultats sont présentés sur la Figure 5.

Légende de la Figure 5 :

La Figure 5A représente une coloration au Bleu de Coomassie d'un gel d'électrophorèse obtenu à partir d'extraits protéiques d'*E. Coli* contenant l'ADNc de PIMT-1, PIMT-2 ou pas d'ADNc (T-) dans le vecteur d'expression pDest17. Les flèches indiquent la présence de la protéine PIMT *d'Arabidopsis thaliana.*

La Figure 5B représente un Western blot réalisé avec l'anticorps 1 sur les protéines PIMT-1 et PIMT-2 purifiées à partir des extraits présentés à la Figure 6A.

Ces résultats montrent que l'anticorps 1 permet de révéler spécifiquement la protéine PIMT-1 *d'Arabidopsis thaliana.*

## EXEMPLE 4 : DETERMINATION PAR TRANSFERT DE WESTERN DE LA TENEUR EN PROTEINE IAMT DANS LES GRAINES SECHES D'*A. THALIANA,* ET DE SON EVOLUTION AU COURS DU VIELLISSEMENT.

Les anticorps anti-IAMT générés, comme décrit à l'Exemple 3 ci-dessus, contre le peptide 1 (anticorps 1) ont été utilisés pour déterminer la teneur en protéine L-isoaspartyl méthyltransférase de graines sèches d'*Arabidopsis thaliana* de type sauvage et de type mutant fraîchement récoltées ou vieillies pendant 2, 6 et 9 semaines comme décrit à l'Exemple 2 ci-dessus.

## Extraction des protéines des graines :

Les graines sont broyées dans l'azote liquide jusqu'à l'obtention d'une fine poudre. La poudre est transférée au tiers d'un tube EPPENDORF de 2 ml complété avec du tampon de LAEMMLI 2X (LAEMMLI U.K., *Nature,* 1970, 227: 680) préalablement chauffé à 100˚C. Les échantillons sont incubés à 100˚C durant 5 minutes avant d'être centrifugés à 4˚C (6000g, 5 minutes). Le surnageant est récupéré, aliquoté et conservé à -20˚C. Préalablement à l'utilisation, 100 µl de surnageant sont précipités dans un volume équivalent d'acide trichloracétique à 20% (13000 rpm, 5 minutes). Le culot est lavé avec 200 µl d'acétone 80%, séché et repris dans 1 ml de soude 0.2N. La concentration est déterminée par mesure de l'absorbance à 595 nm du complexe protéine-réactif de Bradford (Bio-Rad). La sérum albumine bovine est utilisée pour obtenir une courbe étalon.

**Transfert de Western :**

L'électrophorèse des protéines (10 µg) est conduite en conditions dénaturantes (SDS 0.1%) sur un gel de polyacrylamide (12%) conformément à LAEMMLI *(Nature,* 1970, 227: 680). Le transfert semi-sec est effectué sur une membrane de nylon de type HYBOND C (AMERSHAM) sous un courant de 20V pendant 50 minutes. La membrane est ensuite incubée 40 minutes dans une solution de PBS-T (NaCl, 1,4 M; KCI, 27 mM; $Na_2HPO_4$, 81 mM ; $KH_2PO_4$, 150 mM ; Tween® 20, 0,001 %) à laquelle est ajouté du lait en poudre (5%) afin de saturer les sites non spécifiques.

L'anticorps primaire anti-IAMT est ajouté (dilution 1/5000ème) et l'incubation est réalisée pendant la nuit à 4˚C sous agitation. Après 3 lavages de 10 minutes avec du PBS-Tween, la membrane est incubée avec du PBS-Tween® + lait additionné de l'anticorps secondaire anti-Ig G de lapin couplé à la peroxydase (Bio-Rad), durant 2 heures à température ambiante sous agitation. La membrane est à nouveau lavée 2 fois avec du PBS-Tween. La révélation est effectuée par chimioluminescence, en utilisant le kit COVALIGHT, dans les conditions préconisées par le fabricant (CovalAb). Après 1 minute, la membrane est transférée sous un film autoradiographique et exposée 30 minutes.

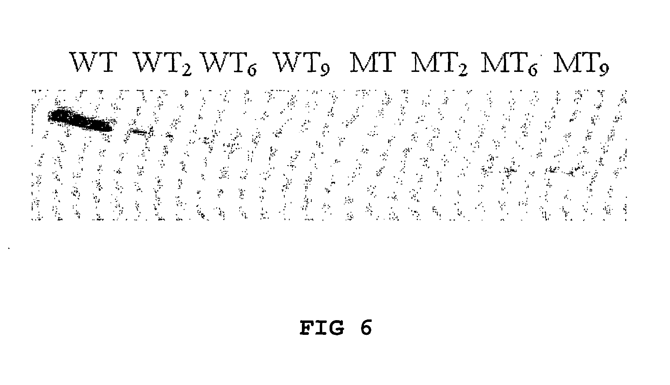

Les résultats sont illustrés par la Figure 6.
Légende de la Figure 6 : graines d'*Arabidopsis thaliana* sauvage (WT) ; graines du mutant *atpcm1.1⁻* (MT) ; 2, 6, ou 9 en indice : graines vieillies 2, 6 ou 9 semaines.

Dans tous les cas, une seule bande est observée. La protéine reconnue correspond à la taille attendue pour l'enzyme PIMT-1, ce qui montre la spécificité de l'anticorps 1 pour cette enzyme dans la graine sèche.

On observe chez les graines de type sauvage, une diminution de la quantité de protéine L-isoaspartyl méthyltransférase, qui se manifeste dès 2 semaines de vieillissement accéléré, et qui se poursuit jusqu'à la fin de l'expérimentation. Au contraire, dans les graines du mutant *atpcm1.1⁻* on observe une augmentation de la quantité d'enzyme au cours du vieillissement.

Cette observation corrobore les résultats rapportés à l'Exemple 1, montrant l'accumulation du transcrit spécifiques du gène PIMT-1 dans les graines sèches du mutant *atpcm1.1⁻*, et peut être corrélée avec la meilleure vigueur germinative du mutant *atpcm1.1⁻* après vieillissement accéléré rapportée à l'Exemple 2.

Ces données apportent la preuve génétique que l'accumulation de la protéine PIMT-1 est associée à une meilleur longévité de la semence, et indiquent que la protéine L-isoaspartyl méthyltransférase constitue un marqueur intéressant de l'évolution de la vigueur germinative d'un lot de semences au cours du stockage.

**EXEMPLE 5: UTILISATION DE L'ANTICORPS ANTI-IAMT POUR DETERMINER L'APTITUDE A LA CONSERVATION DE SEMENCES DE MAÏS, DE COLZA, DE TOMATE ET DE HARICOT.**

Des expérimentations complémentaires ont été effectuées afin de vérifier si l'anticorps obtenu contre la L-isoaspartyl méthyltransférase *d'Arabidopsis thaliana* reconnaissait également les enzymes orthologues d'autres espèces végétales, et si l'IAMT constituait également chez ces espèces un marqueur de l'aptitude à la germination.

Plusieurs espèces d'intérêt agronomique ont été choisies pour ces expérimentations afin d'explorer un spectre large de plantes cultivées : le maïs est un représentant des monocotylédones et chez les dicotylédones le haricot est une légumineuses, le colza une crucifère et la tomate une solanacée.

Le vieillissement accéléré est réalisée par incubation à une température donnée après avoir équilibré les graines à une teneur en eau souhaitée (16 à 17% généralement).

Les graines sont d'abord sur-séchées à 20% HR (- 28˚C) en étuve ventilée. Pour déterminer la teneur en eau, 10 g de graines sont grossièrement broyés et pesés avant d'être chauffés à sec (130˚C, 1 heure). La poudre de graines est refroidie sous vide et repesée (la précision est au dix millième de gramme). On peut ensuite estimer la teneur en eau initiale des graines après sur-séchage selon la formule :

$$\frac{M_i - M_f}{M_i} = TE_i$$

$M_i$ = masse de graines avant chauffage
$M_f$ = masse de graines après chauffage
$TE_i$ = teneur en eau initiale

Afin de déterminer la masse d'eau à ajouter à une masse de graines connue pour fixer sa teneur en eau à la valeur souhaitée, on utilise la formule suivante :

$$\frac{M_1(TE_i - TE_f)}{TE_f - 1} = M_2$$

$TE_i$ = teneur en eau initiale
$TE_f$ = teneur en eau finale
$M_1$ = masse de graines
$M_2$ = masse d'eau finale

Les graines sont placées dans un récipient étanche contenant le volume d'eau calculé. L'imbibition est réalisée pendant 3 jours sous agitation.

**Vieillissement chez le haricot :**

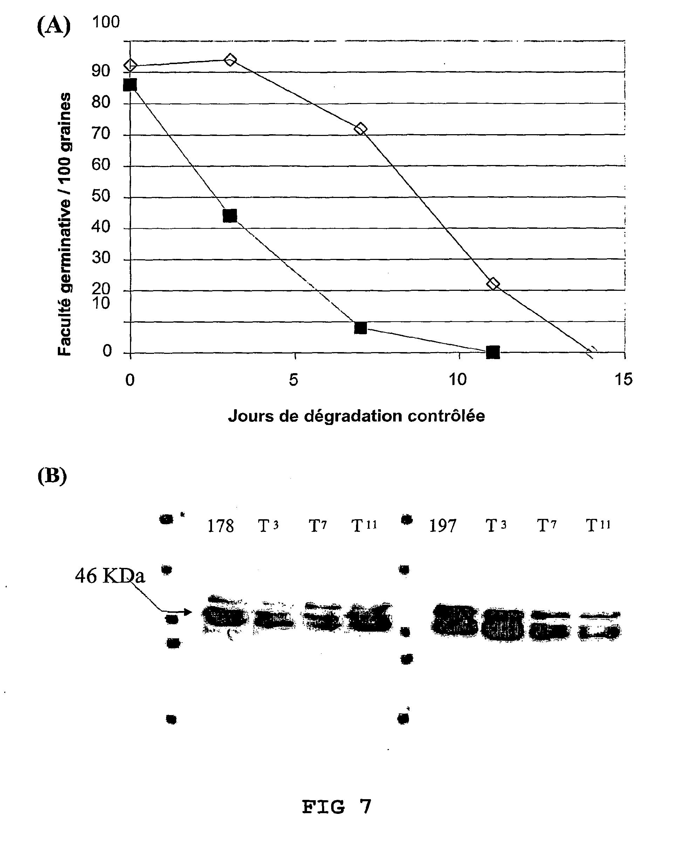

2 lots de semences de haricot (récoltes 2000 = lot 178 et 2001 = lot 197) sont équilibrés à 16% de teneur en eau et placés en bocaux hermétiques à 40°C. Un échantillon est prélevé au bout de 3, 7, 11 et 14 jours de vieillissement et un test de germination est effectué sur 50 graines.
Les résultats sont illustrés par la Figure 7A.
Légende de la Figure 7A : Faculté germinative, exprimée pour 100 graines, en fonction de la durée de la dégradation contrôlée.

◇: lot 197
■ : lot 178

Ces résultats montrent que les 2 lots ne se comportent pas de la même manière dans des conditions semblables de dégradation puisque le lot 178 (récolte de 2000) atteint 45% de germination au terme de 3 jours de vieillissement alors que ce pourcentage de germination est atteint en 9 jours pour le lot 197 (récolte de 2001).
Les protéines issues des graines sèches de haricot, provenant des récoltes de 2000 et de 2001, ainsi que des graines vieillies artificiellement à partir de ces 2 récoltes, ont également été caractérisées en Western blot à l'aide de l'anticorps 1.
Les résultats sont illustrés par la Figure 7B.
Légende de la Figure 7B : 178 (récolte de 2000) ; 197 (récolte de 2001) ; (T3), (T7), (T11) jours : 3, 7, et 11 jours de vieillissement.
L'anticorps 1 met en évidence 2 ou 3 protéines d'une taille d'environ 46 kDa. On observe que le lot 197, qui présente une meilleure aptitude à la conservation en vieillissement accéléré que le lot 178, contient une plus importante quantité de protéines reconnues par l'anticorps 1. En vieillissement accéléré, le lot 197 (récolte 2001) montre une baisse de la concentration de cet ensemble de protéines pour les temps de vieillissement qui précède la perte de viabilité. Le lot 178 (récolte 2000), dont la capacité germinative a chuté dès le 3ème jour de vieillissement, ne présente pas de diminution sensible de la quantité de protéines reconnues par l'anticorps 1.

**Vieillissement chez le colza :**

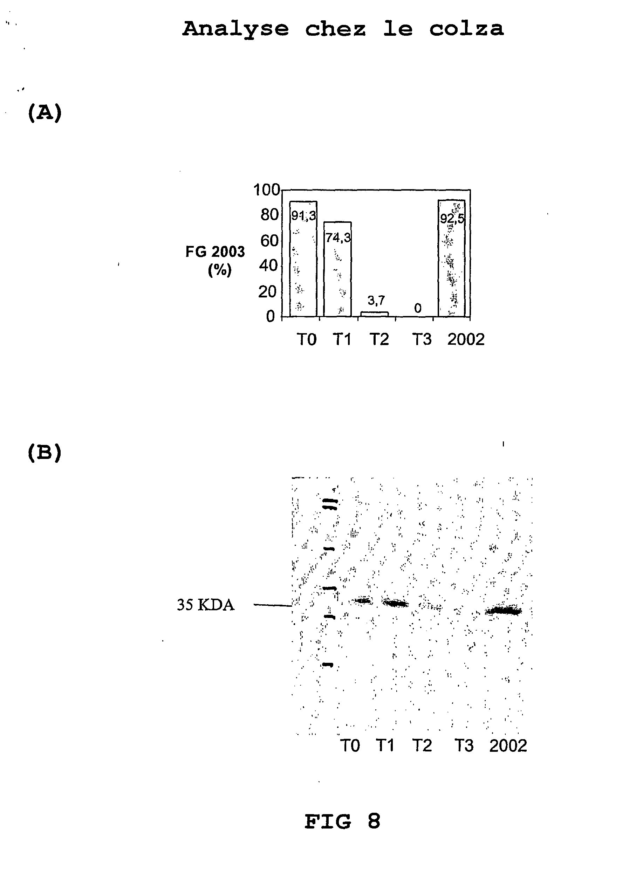

Le lot de semence (colza Ontario, récolte 2002) est soumis à un traitement de vieillissement accéléré dans les conditions décrites ci-dessus sur le haricot. Les échantillons sont prélevés de la même manière aux mêmes temps de vieillissement et les facultés germinatives (FG) des différents échantillons sont illustrées à la Figure 8A.
Légende de la Figure 8A :

Faculté germinative (FG), exprimée pour 100 graines, en fonction de la durée vieillissement. Les semences matures sèches d'une récolte de 2002 ont une faculté germinative de 92 % en 2003, et la faculté germinative de ces graines diminue à 91%, 74%, 3%, et 0% au cours des temps T0, T1, T2 et T3 du traitement de vieillissement respectivement.

Les protéines issues des graines sèches de colza, provenant de la récolte de 2002, ainsi que des graines vieillies artificiellement ont également été caractérisées en Western blot à l'aide de l'anticorps 1.
Les résultats sont illustrés par la Figure 8B.
Légende de la Figure 8B :

L'anticorps 1 met en évidence 1 protéine d'une taille d'environ 32 kDa. On observe que le lot de graines n'ayant pas subi de traitement de vieillissement, qui présente une meilleure faculté germinative, contient une plus importante quantité de protéines reconnues par l'anticorps 1. Au cours des temps T0,T1, T2 et T3 de vieillissement accéléré, ce lot montre une baisse de la concentration de cette de protéines qui est proportionnelle à la baisse de faculté germinative représentée à la Figure 8A.

## Vieillissement chez la tomate.

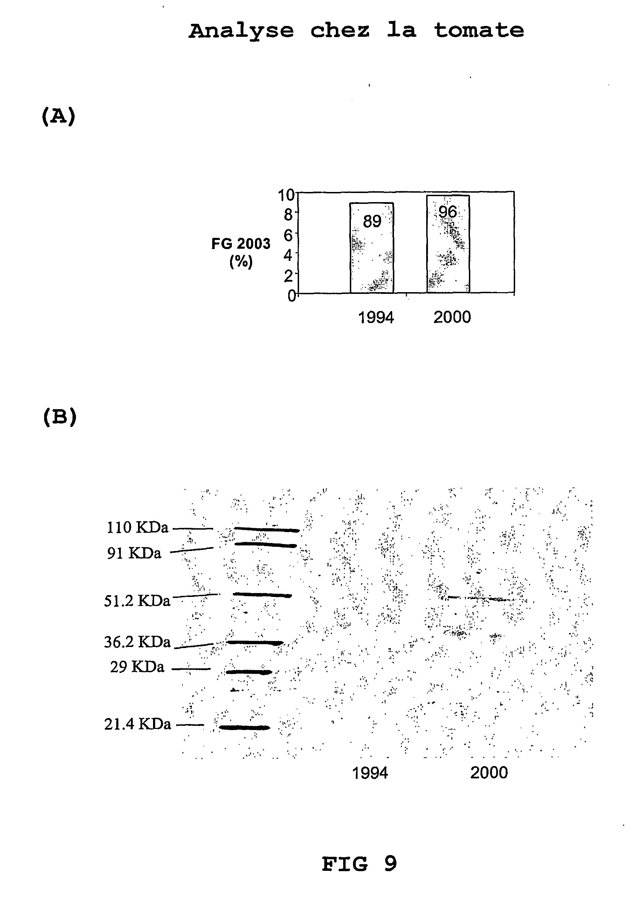

Deux lots de semences de tomate (variété PicNic) d'années de récoltes différentes (1994 et 2000) ont été analysés en 2003 pour leur faculté germinatives (illustrée Figure 9A). Ces 2 lots de semences ont donc subi un vieillissement de 9 ans et de 3 ans respectivement dans des conditions de conservation optimales telles que celles utilisées par les semenciers classiquement.
Légende de la Figure 9A :

Faculté germinative (FG) en 2003, exprimée pour 100 graines, en fonction de l'année de récolte (1994 et 2000). Les lots de semence récoltés en 1994 et en 2000 ont une faculté germinative de 89% et 96% respectivement.

Les protéines issues des graines sèches de tomate, provenant de ces 2 années de récolte de 2002, ont également été caractérisées en 2003 en Western blot à l'aide de l'anticorps 1.
Les résultats sont illustrés par la Figure 9B.
Légende de la Figure 9B :

L'anticorps 1 met en évidence 3 ou 4 protéines d'une taille d'environ 24 KDa, 40 kDa et 52 KDa. On observe que le lot récolté en 2000, qui présente une meilleure FG, contient une plus importante quantité de protéines reconnues par l'anticorps 1.

## Vieillissement chez le maïs :

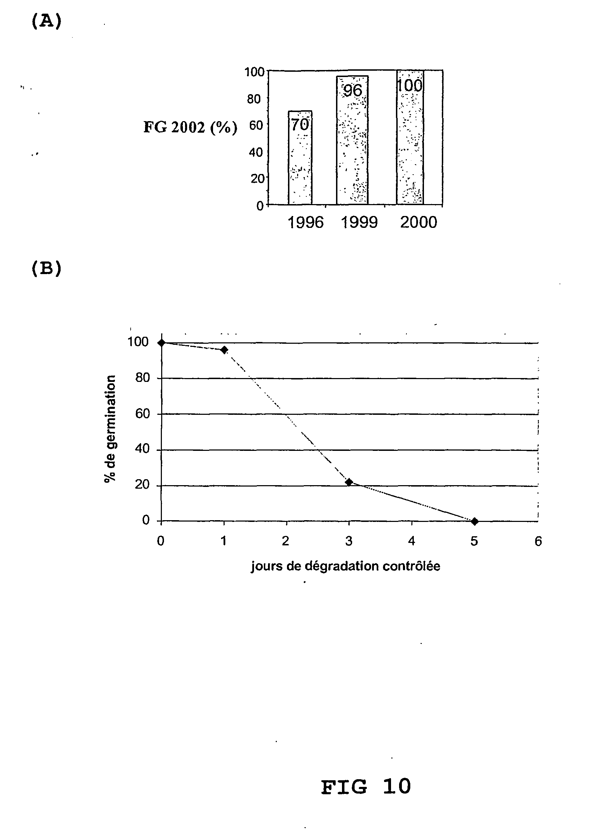

Les facultés germinatives de lots de semences issues de différentes années de récoltes (1996, 1999, 2000, 2001) ont été analysées en 2002.
Les résultats de cette analyse sont illustrés sur la Figure 10A.
Légende de la Figure 10A :

Faculté germinative (FG) en 2002, exprimée pour 100 graines, en fonction de l'année de récolte (1996, 1999, 2000 et 2001). Dans les conditions de conservation classique, les lots de semences de maïs récoltés en 2001 et 2000 germent à 100 %, le lot de semences récolté en 1999 germe à 96 % et le lot de semences récolté en 1996 germe à 75 %.

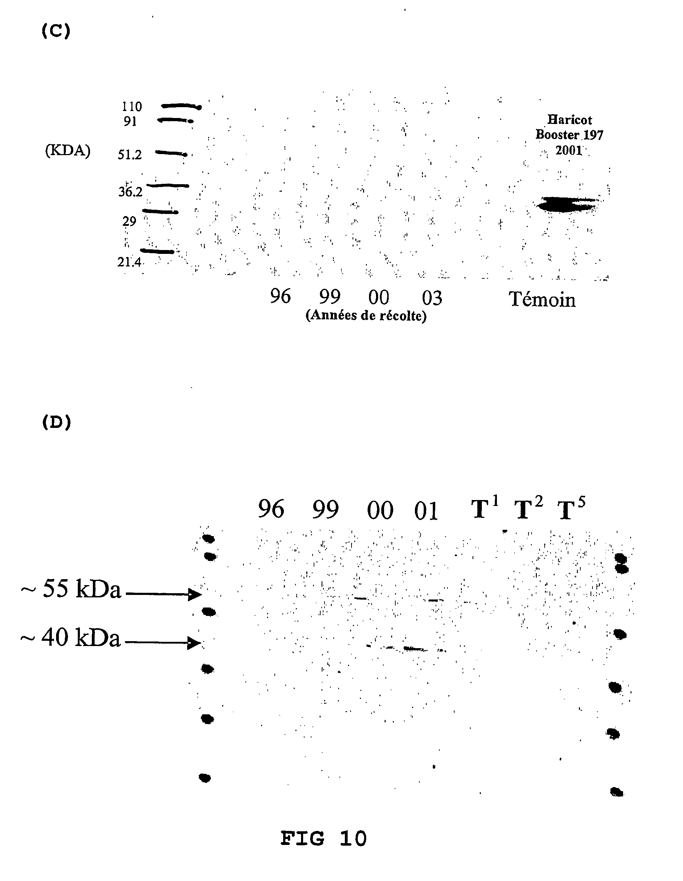

Afin de réaliser la dégradation contrôlée du maïs, la teneur en eau de la graine est fixée à 17%. Le vieillissement est conduit à 45˚C. Un échantillon est prélevé au bout de 1, 3 et 5 jours de vieillissement et un test de germination est effectué sur 200 graines.
Dans ces conditions, on obtient une incapacité totale à germer en 5 jours, comme le montre la Figure 10B.
Légende de la Figure 10B : Pourcentage de germination en fonction de la durée de la dégradation contrôlée.
Les protéines des graines sèches issues des récoltes de 1996, 1999, 2000, et 2001, ainsi que celles de graines récoltées en 2001 et vieillies artificiellement, ont été extraites en 2003 et analysées en transfert de Western en utilisant l'anticorps 1 et l'anticorps dirigé contre le peptide 2 (anticorps 2) comme décrit à l'Exemple 4 ci-dessus. Les résultats apparaissent sur la Figure 10C (pour l'anticorps 1) et 10D (pour l'anticorps 2). Légende des Figures 10C et 10D : 96, 99, 00, 01 : année de récolte ; (T1), (T3), (T5) jours : 1, 3, et 5 jours de vieillissement.
Dans la Figure 10C aucun signal n'est détecté avec l'anticorps 1 sur les extraits protéique de maïs, comparés aux signaux obtenus sur l'extrait protéique de haricot utilisé comme témoins. Ce résultat indique que l'anticorps 1 ne permet pas de détecter la protéine PIMT chez le maïs. Un même résultat (non montré ici) a été obtenu chez d'autres monocotylédones telles que le blé et l'oignon. L'anticorps 1 montre une spécificité vis à vis des dicotylédones.
L'utilisation de l'anticorps 2 (Figure 10D) permet par contre de détecter 2 protéines de poids moléculaires 40 kDa et 55 kDa chez le maïs. Ces protéines ne sont pas détectables dans les graines récoltées en 1996, pour lesquelles le pourcentage de germination, mesuré parallèlement, est de 75%. La teneur en protéine de 40 kDa est maximale dans les graines issues de la récolte de 2001, puis elle décroît ensuite avec l'âge de la récolte. Dans les grains de 1999, elle est à peine détectable, alors que le pourcentage de germination de ces graines, est encore de 96%. La teneur en protéine de 55 kDa demeure au contraire relativement stable entre les récoltes de 1999 et de 2001.
En vieillissement accéléré, la quantité de protéine de 55 kDa demeure stable pendant le premier jour de vieillissement accéléré, puis elle n'est plus détectable. La protéine de 40 kDa n'est plus détectable dès le premier jour de vieillissement accéléré. Les mêmes observations (résultats non montrés) ont été faites chez le blé et l'oignon).

Il apparaît donc que la disparition des protéines de 40 et 55 kDa reconnues par l'anticorps 2 précède le déclin de la capacité germinative. Ces protéines, et notamment la protéine de 40 kDa, sont donc utilisables en tant que marqueur prédictif de vieillissement du grain. Cette analyse montre également que l'anticorps 2 permet de suivre l'évolution de cette protéine aussi chez les monocotylédones alors que l'anticorps 1 semble lui plus spécifique des dicotylédones.

Ces résultats montrent que des anticorps anti-IAMT conformes à l'invention permettent de mettre en évidence des différences d'aptitude à la conservation entre lots de semences, aussi bien dans des conditions de conservation naturelle, telles que celles utilisées par les semenciers, qu'en situation de détérioration contrôlée.

**Revendications**

1. Procédé de détermination de la vigueur germinative et/ou de l'aptitude d'un lot de semences à la conservation, **caractérisé en ce qu'**il comprend la quantification sur un échantillon de semences prélevé sur ledit lot, des protéines reconnues par des anticorps anti-L-isoaspartyl méthyltransférase dirigés contre une région de ladite protéine définie par la séquence (I) : RYVPLTSRX$_1$X$_2$QLX$_3$ (SEQ ID NO : 1), dans laquelle X$_1$ représente E, V ou S, X$_2$ représente A ou E, X$_3$ représente R, G ou Q.

2. Procédé selon la revendication 1, **caractérisé en ce que** l'on met en oeuvre un anticorps anti-L-isoaspartyl méthyltransférase dirigé contre un peptide choisi parmi : le peptide de séquence RYVPLTSREAQLR (SEQ ID NO : 5), le peptide de séquence RYVPLTSRVEQLG (SEQ ID NO : 6), le peptide de séquence RYVPLTSRSAQLQ (SEQ ID NO : 7).

3. Anticorps anti-L-isoaspartyl méthyltransférase, tel que défini dans une quelconque des revendications 1 ou 2.

4. Procédé de quantification de la L-isoaspartyl méthyltransférase dans du matériel végétal, **caractérisé en ce qu'**il comprend la mise en contact dudit matériel avec un anticorps anti-L-isoaspartyl méthyltransférase selon la revendication 3.

5. Utilisation d'un anticorps anti-L-isoaspartyl méthyltransférase selon la revendication 3 pour déterminer la vigueur germinative et/ou l'aptitude à la conservation d'un lot de semences.

## Claims

1. Method for determining the germination vigour and/or the storability of a seed batch, **characterised in that** it comprises quantifying, on a sample of seeds taken from said batch, proteins recognised by anti-L-isoaspartyl methyltransferase antibodies directed against a region of said protein defined by sequence (I): RYVPLTSRX$_1$X$_2$QLX$_3$ (SEQ ID NO: 1), wherein X$_1$ represents E, V or S, X$_2$ represents A or E, X$_3$ represents R, G or Q.

2. Method according to claim 1, **characterised in that** use is made of an anti-L-isoaspartyl methyltransferase antibody directed against a peptide selected from: the peptide of sequence RYVPLTSREAQLR (SEQ ID NO: 5), the peptide of sequence RYVPLTSRVEQLG (SEQ ID NO: 6), the peptide of sequence RYVPLTSRSAQLQ (SEQ ID NO: 7).

3. Anti-L-isoaspartyl methyltransferase antibody as defined in either claim 1 or claim 2.

4. Method for quantifying L-isoaspartyl methyltransferase in plant material, **characterised in that** it comprises bringing said material into contact with an anti-L-isoaspartyl methyltransferase antibody according to claim 3.

5. Use of an anti-L-isoaspartyl methyltransferase antibody according to claim 3 for determining the germination vigour and/or the storability of a seed batch.

## Patentansprüche

1. Verfahren zur Bestimmung der Keimungskraft und/oder der Eignung zur Konservierung einer Partie von Samen, **dadurch gekennzeichnet, dass** es die quantitative Bestimmung der Proteine, die durch Antikörper gegen L-Isoaspartylmethyltransferase erkannt werden, die gegen eine Region des genannten Proteins gerichtet sind, die durch die Sequenz (I): RYVPLTSRX$_1$X$_2$QLX$_3$ (SEQ ID NO: 1), worin X$_1$ E, V oder S darstellt, X$_2$ A oder E darstellt, X$_3$ R, G oder Q darstellt, definiert wird, an einer Probe von Samen, die aus der genannten Partie entnommen wurde, umfasst.

2. Verfahren nach Anspruch 1, **dadurch gekennzeichnet, dass** man einen Antikörper gegen L-Isoaspartylmethyltransferase verwendet, der gegen ein Peptid gerichtet ist, das ausgewählt ist aus: dem Peptid der Sequenz RYVPLTSREAQLR (SEQ ID NO: 5), dem Peptid der Sequenz RYVPLTSRVEQLG (SEQ ID NO: 6), dem Peptid der Sequenz RYVPLTSRSAQLQ (SEQ ID NO: 7).

3. Antikörper gegen L-Isoaspartylmethyltransferase, wie er in einem der Ansprüche 1 oder 2 definiert ist.

4. Verfahren zur quantitativen Bestimmung der L-Isoaspartylmethyltransferase in pflanzlichem Material, **dadurch gekennzeichnet, dass** es die Verwendung des Materials mit einem Antikörper gegen L-Isoaspartylmethyltransferase nach Anspruch 3 umfasst.

5. Verwendung eines Antikörpers gegen L-Isoaspartylmethyltransferase nach Anspruch 3, um die Keimungskraft und/oder die Eignung zur Konservierung für eine Partie von Samen zu bestimmen.

FIG 1

FIG 2

**(A)**

Sonde IAMT                Sonde EF

**(B)**

FIG 3

(A)

(B)

FIG 4

**(A)**

ADNc ADNc
PIMT-1 PIMT-2 T-

**(B)**

PIMT-1
(10 µg)    PIMT-2 (10 µg)    PIMT-1 (20 µg)    PIMT-2 (20 µg)

(anticorps 1)

FIG 5

WT WT$_2$ WT$_6$ WT$_9$ MT MT$_2$ MT$_6$ MT$_9$

FIG 6

**(A)**

Jours de dégradation contrôlée

**(B)**

FIG 7

# Analyse chez le colza

**(A)**

FG 2003 (%)

100, 80, 60, 40, 20, 0 — T0 (91,3) T1 (74,3) T2 (3,7) T3 (0) 2002 (92,5)

**(B)**

35 KDA

T0  T1  T2  T3  2002

**FIG 8**

# Analyse chez la tomate

**(A)**

**(B)**

FIG 9

(A)

FG 2002 (%)

(B)

jours de dégradation contrôlée

FIG 10

**(C)**

**(D)**

FIG 10

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• FR 0313858

**Littérature non-brevet citée dans la description**

• **WALTERS C.** *Seed Sci. Res.,* 1998, vol. 8, 223-244
• **WALTERS C.** *Seed Sci. Res.,* 1998, vol. 8, 223-24 4
• **O'CONNER C.M et al.** *Biochem. Biophys. Res.,* 1985, vol. 132, 1144-1150
• **TRIVEDI L. et al.** *Eur. J. Biochem.,* 1982, vol. 128, 349-354
• **MUDGETT M.B. et al.** *Biochemistry,* 1993, vol. 32, 11100-11111
• *J. Biol. Chem.,* 1994, vol. 269 (41), 25605-25612
• **SHEN-MILLER et al.** *Am. J. Bot.,* 1995, vol. 82 (11), 1367-1380
• **MUDGETT M.B. et al.** *Plant. Mol. Biol.,* 1996, vol. 30, 723-737
• **THAPAR N. et al.** *Protein Expr. Purif.,* 2000, vol. 20, 237-251
• **MUDGETT M.B. et al.** *Plant Physiol.,* 1997, vol. 115, 1481-1489
• **KESTER et al.** *J. Exp. Bot.,* 1997, vol. 309, 943-949
• **THAPAR N. et al.** *Plant Physiol,* 2001, vol. 125 (2), 1023-1035
• **CLARKE S.** *Int. J. Pept. Protein Res.,* 1987, vol. 30, 808-821
• **XU Q. et al.** *Plant Physiol.,* 2004, vol. 136, 1-13
• **VERWOERD et al.** *Nucleic Acids Res.,* 1989, vol. 17 (6), 2362